# EUROPÄISCHE PATENTSCHRIFT

(11) **EP 0 596 381 B1**
(45) Veröffentlichungstag und Bekanntmachung des Hinweises auf die Patenterteilung: **18.08.1999**
(21) Anmeldenummer: 93117299.3
(22) Anmeldetag: 26.10.1993
(51) Int. Cl.: C07D 323/06

(54) **Verfahren zum Abtrennen von Trioxan aus einem wässrigen Gemisch**
Process for the separation of trioxane from aqueous mixture
Procédé de séparation de trioxanne d'un mélange aqueux

(30) Priorität: 31.10.1992 DE 4236853
(43) Veröffentlichungstag der Anmeldung: 11.05.1994
(73) Patentinhaber: Ticona GmbH, 65451 Kelsterbach (DE)
(72) Erfinder: Arnold, Dieter, D-61462 Königstein/Taunus (DE)

(56) Entgegenhaltungen:
- EP-A- 0 133 669

## Beschreibung

Bei der Herstellung von Trioxan entsteht ein azeotropes Gemisch, das im wesentlichen aus Trioxan, Wasser und Formaldehyd besteht. Aus diesem Gemisch wird Trioxan extraktiv mit Hilfe eines Schleppmittels, wie z.B. Methylenchlorid oder Benzol abgetrennt. In einer nachfolgenden Destillation wird das Schleppmittel zurückgewonnen und der Extraktivdestillation wieder zugeführt. Bei diesem Verfahren müssen große mengen Schleppmittel eingesetzt und mit hohem Energieaufwand zurückgewonnen werden. Zwangsweise anfallende Emissionen müssen aufwendig entsorgt werden, da Methylenchlorid und Benzol als gefährliche Schadstoffe eingestuft sind.

Hier will die Erfindung Abhilfe schaffen. Die Erfindung löst die Aufgabe dadurch, daß man dem Gemisch Wasser durch Pervaporation entzieht und das an Wasser verarmte Gemisch (Retentat) durch Rektifikation in Trioxan und ein azeotropes Gemisch aus Trioxan, Wasser und Formaldehyd zerlegt.

Die Pervaporation kann bei Temperaturen von 70 bis 120°C mit einer Membran aus Polyvinylalkohol bei Drücken von 1 bis 3 bar auf der Retentatseite und 30 bis 150 mbar auf der Permeatseite durchgeführt werden.

Die Vorteile des erfindungsgemäßen Verfahrens sind im wesentlichen darin zu sehen, daß keine zusätzliche Komponente (Schleppmittel) erforderlich ist, somit das Emissionsproblem entfällt und der Energiebedarf verringert wird (niedrigeres Temperaturniveau). Darüber hinaus erfordert die Pervaporationseinheit ein geringeres Bauvolumen als die Extraktivdestillation bei vergleichbarem Durchsatz.

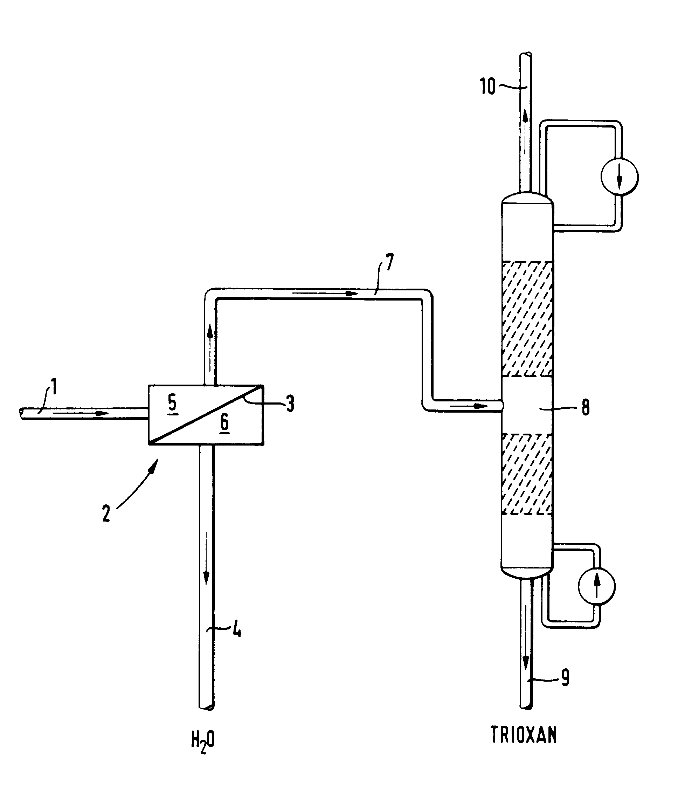

Die Erfindung wird anhand der Figur in beispielsweiser Ausführung im folgenden näher erläutert.

Einem wäßrigen Gemisch (Leitung 1) bestehend aus 65 Gew.-% Trioxan, 27,5 Gew.-% Wasser und 7,5 Gew.-% Formaldehyd wird in einer Pervaporationseinheit 2, die eine Membran 3 aus Polyvinylalkohol der Firma GFT (Gesellschaft für Trenntechnik mbH, D-6680 Neunkirchen/Saar) enthält, Wasser (Leitung 4) entzogen. Die Trennung erfolgt bei 90°C. Der Druck auf der Retentatseite 5 beträgt 1 bar, auf der Permeatseite 6 50 mbar. Man erhält als Permeat Wasser und als Retentat ein Gemisch aus 84 Gew.-% Trioxan, 10 Gew.-% Formaldehyd und 6 Gew.-% Wasser (Leitung 7). Das Retentat wird in einer Rektifikationskolonne 8 unter Normaldruck in reines Trioxan (Sumpfprodukt, Leitung 9) und ein azeotropes Gemisch aus Trioxan, Formaldehyd und Wasser (Kopfprodukt, Leitung 10) zerlegt.

## Patentansprüche

1. Verfahren zum Abtrennen von Trioxan aus einem wäßrigen Gemisch, das im wesentlichen aus Trioxan, Wasser und Formaldehyd besteht, dadurch gekennzeichnet, daß man dem Gemisch Wasser durch Pervaporation entzieht und das an Wasser verarmte Gemisch (Retentat) durch Rektifikation in Trioxan und ein azeotropes Gemisch aus Trioxan, Wasser und Formaldehyd zerlegt.

2. Verfahren nach Anspruch 1, dadurch gekennzeichnet, daß die Pervaporation mit einer Membran aus Polyvinylalkohol bei einer Temperatur von 70 bis 120°C und einem Druck von 1 bis 3 bar auf der Retentatseite und 30 bis 150 mbar auf der Permeatseit durchgeführt wird.

## Claims

1. A process for separating trioxane from an aqueous mixture consisting essentially of trioxane, water and formaldehyde, which comprises extracting water from the mixture by pervaporation and separating the water-depleted mixture (retentate) by rectification into trioxane and an azeotropic mixture of trioxane, water and formaldehyde.

2. The process as claimed in claim 1, wherein pervaporation is carried out using a membrane of polyvinyl alcohol at a temperature of from 70 to 120°C and a pressure of from 1 to 3 bar on the retentate side and from 30 to 150 mbar on the permeate side.

## Revendications

1. Procédé de séparation de trioxanne à partir d'un mélange aqueux qui est constitué essentiellement de trioxanne, d'eau et de formaldéhyde, caractérisé en ce que l'on extrait du mélange de l'eau par pervaporation et en ce que l'on décompose le mélange appauvri en eau (rétentat) par rectification dans du trioxanne et un mélange azéotrope constitué de trioxanne, d'eau et de formaldéhyde.

2. Procédé suivant la revendication 1, caractérisé en ce que l'on réalise la pervaporation avec une membrane de poly(alcool de vinyle) à une température de 70 à 120°C et à une pression de 1 à 3 bars du côté du rétentat et de 30 à 150 mbars du côté du perméat.
